# EUROPÄISCHE PATENTANMELDUNG

(11) **EP 0 788 797 A1**
(43) Veröffentlichungstag der Anmeldung: **13.08.1997**
(21) Anmeldenummer: 97101871.8
(22) Anmeldetag: 06.02.1997
(51) Int. Cl.: A61K 31/785, A61K 31/155

(54) **Verwendung von PHMB zur Behandlung von durch sich intrazellulär vermehrende Erreger verursachten Infektionen**

(30) Priorität: 08.02.1996 DE 19604475
(71) Anmelder: Fresenius AG, 61350 Bad Homburg v.d.H (DE)
(72) Erfinder: Kirschner, Ulrich, Dr., 61350 Bad Homburg (DE); Jethon, Frank, 61350 Bad Homburg (DE); Kramer, Axel, 17489 Greifswald (DE)
(74) Vertreter: Fuchs, Luderschmidt & Partner

(57) **Zusammenfassung**

Gegenstand ist die Verwendung von Poly(hexamethylen)biguanid (PHMB) mit einer mittleren Molekulargewichtsverteilung bis 5.000 zur Behandlung von durch sich intrazellulär vermehrende Erreger verursachten Infektionen. Sich intrazellulär vermehrende Erreger umfassen sowohl Bakterien als auch Viren, wobei spezielle Beispiele für Bakterien Chlamydien, wie Chlamydia trachomatis und Chlamydia psittaci, und Neisserien, wie Neisseria gonorrhoeae und Neisseria meningitidis sind. Die Verwendung ist insbesondere auf die Behandlung von Infektionen im Auge, im Urogenitaltrakt sowie im Peritonealraum gerichtet.

## Beschreibung

Gegenstand der vorliegenden Erfindung ist die Verwendung von Poly(hexamethylen)biguanid zur Behandlung von durch sich intrazellulär vermehrende Erreger verursachten Infektionen.

Aus dem Stand der Technik ist bekannt, daß Poly(hexamethylen)biguanid (PHMB) eine bakterizide und fungizide Wirkung aufweist (vgl. z.B. GB-PS 1,202,495). PHMB wird daher in vielen Bereichen als Desinfektionsmittel, z.B. in Form von Lösungen oder Spray's eingesetzt. Anwendung finden sie z.B. in der Lebensmittelindustrie zur Reinigung und Desinfektion von Räumen und Geräten, zur Stabilisierung von Getränken und zur Reinigung und Stabilisierung von Wasser, z.B. auch in Schwimmbädern zur Bekämpfung von Algen- und Bakterienwuchs. Aus der DE-OS 35 37 627 ist bekannt, daß durch Kombination von PHMB mit einem Molekulargewicht von 1.700 bis 2.500 mit einer geringen Menge Polyethylenglycol Desinfektionsmittel erhalten werden, die auch als lokale Antiseptika in der Wundbehandlung eingesetzt werden. Als PHMB geeignet ist gemäß dieser DE-OS z.B. das PHMB, das als Hydrochlorid unter dem Warenzeichen Vantocil® IB von der ICI vertrieben wird.

In der EP 0 4 50 117 wird eine Ringerlösung und deren Anwendung als bakterizid wirkendes lokales Wundbehandlungsmedikament beschrieben, wobei die lactatfreie Ringerlösung zusätzlich 0,1 % bis 0,2 % eines Konzentrates gelöst enthält, das aus einer 20 %igen wässrigen Poly(hexamethylen)biguanid-hydrochlorid-Lösung besteht, in der pro 100 ml 1 g Polyethylenglycol mit einem Molekulargewicht von etwa 4.000 gelöst ist. Als PHMB wird ebenfalls lediglich das unter dem Warenzeichen Vantocil® IB der Firma ICI vertriebene Produkt als geeignet beschrieben. Unter dem Warenzeichen Lavasept® ist ein Produkt für die Wundheilung bekannt, wobei das Lavasept-Konzentrat eine wässrige Lösung von 20 Gew.-% PHMB und 1 Gew.-% Polyethylenglycol 4.000 darstellt und das PHMB das Handelsprodukt Vantocil® IB der Firma ICI ist.

In der US-PS 4,758,591 werden Lösungen beschrieben, die eine mikrobizid- oder fungizid-wirksame Menge eines Biguanides oder eines wasserlöslichen Salzes desselben in einer Menge von 0,000001 bis 0,0003 Gew.-% enthalten, die für Kontaktlinsen, ophthalmologische Produkte und dermatologische Formulierungen, die nahe des Auges angewandt werden, eingesetzt werden.

Aus der GB-PS 1,432,345 sind Zusammensetzungen für die Verwendung in Verbindung mit Augen und Kontaktlinsen bekannt, die mindestens ein ophthalmisch akzeptables polymeres Biguanid enthalten.

Bei durch sich intrazellulär vermehrenden Erregern verursachten Infektionen handelt es sich durchweg um schwierig zu therapierende Infektionen. Sich intrazellulär vermehrende Erreger umfassen sowohl Bakterien als auch Viren. Spezielle Beispiele für Bakterien sind Chlamydien, wie Chlamydia trachomatis und Chlamydia psittaci, und Neisserien, wie Neisseria gonorrhoeae und Neisseria meningitidis.

Chlamydia trachomatis ist ein typischer Schleimhautparasit, der ausschließlich den Menschen infiziert. Von ihm werden Infektionen wie z.B. das Trachom, Einschlußkonjunktivitis, unspezifische Genitalinfektionen und Lymphogranuloma venereum verursacht. Beim Trachom handelt es sich um eine chronische Keratokonjunktivitis. Die Krankheit kommt in allen Klimazonen vor. Es wird geschätzt, daß mehr als 400 Millionen Menschen an dieser Infektion leiden. 6 Millionen Blinde auf der Welt verdanken ihr Gebrechen dieser Augenkrankheit. Der Erreger wird durch direkten, aber auch indirekten Kontakt übertragen. Die Inkubationszeit ist wegen des häufig schleichenden Beginns nicht sicher anzugeben, kann jedoch mehrere Wochen betragen. Frühsymptome sind Tränenfluß, eitrig-schleimige Sekretion und eine lokale entzündliche Reaktion. Als Therapie kommt der systemische Einsatz von Tetracyclinen über drei Wochen, kombiniert mit einer lokalen Tetracyclinapplikation (4 x täglich 6 Wochen lang) in Frage. Einschlußkonjunktivitis ist eine akute eitrige Papillarkonjunktivitis, die das Neugeborene. aber auch Erwachsene (Schwimmbadkonjunktivitis) befallen kann. Therapeutisch wird die lokale Applikation von Tetracyclinen empfohlen. Chlamydia trachomatis wird heute in 30 bis 60 % der Fälle als ursächlicher Keim der Nichtgonokokken-Urethritis (NGU) des Mannes angesehen. Als Komplikation kann es in diesen Fällen zu Prostatitis und Epididymitis kommen. Chlamydia trachomatitis kann jedoch auch bei der Frau eine symptomatische Urethritis, eine Zervizitis oder auch Salpingitis hervorrvfen. Zur Behandlung dieser unspezifischen Genitalinfektionen wird die Verwendung von Tetracyclinen empfohlen. Lymphogranuloma venereum ist eine nur bei Menschen vorkommende Infektion des Genitales. Die Inkubation beträgt mehrere Tage bis Wochen. An der Infektionsstelle entsteht eine herpitiforme Primärläsion, die oft unbemerkt bleibt. Im weiteren Verlauf bildet sich lokal ein kleines Geschwür sowie eine Lymphadenopathie der regionären Lymphknoten. Die schmerzhaften Lymphknoten schmelzen oft eitrig ein, beim Mann sind Zumeist die inguinalen, bei der Frau die perirektalen Lymphknoten beteiligt. Falls in diesem Stadium keine spezifische Therapie vorgenommen wird, kann der Prozeß in ein chronisches Stadium übergehen und zu einem fibrösen Verschluß der Lymphbahnen führen. Therapeutisch werden Erythromycin, Sulfonamide oder Tetracycline eingesetzt.

Chlamydia psittaci ist der Erreger der Ornithose (Psittacose). Der Mensch infiziert sich im allgemeinen durch Inhalation chlamydienhaltigen Staubs (Vogelkot), seltener durch Inhalation infektiöser Aerosole. Die Erreger gelangen in den tiefen Respirationstrakt, dringen in die Epithelzellen der Bronchiolen ein und vermehren sich dort. Nach einer Infektionszeit von 1 bis 3 Wochen beginnt die Krankheit mit Fieber, Schmerzen und einer atypischen Pneumonie. Als Therapie kommt die Verwendung von Tetracyclinen und Erythromycin in Frage, Sulfonamide sind unwirksam.

Bei Neisseria gonorrhoeae (Gonokokken) handelt es sich um gramnegative, semmelförmige, meist paarig auftretende Kokken. Die Infektion erfolgt, von Ausnahmen abgesehen, durch den Geschlechtsverkehr. Die Gonokokken heften sich mit Haftpili und dem "äußerem Membranprotein" II an den Epithelzellen der Urogenitalschleimhaut an, werden dann durch Phagozytose aufgenommen, vermehren sich in den Epithelzellen, zerstören diese und rufen im subseriösen Gewebe eine akute, eitrige, entzündliche Reaktion hervor. Neben der Urethralschleimhaut können beim Mann Prostata und Nebenhoden mitbefallen sein. Bei der Frau kann der Erreger durch die Cervix in den Uterus, die Tuben und bis in die Peritonealhöhle vordringen und eine Salpingitis oder sogar eine Peritonitis verursachen. Werden die Erreger auf die Konjunktiven übertragen, kann eine eitrige Konjunktivitis entstehen, die vor allem bei Neugeborenen gefürchtet ist. Gonokokken können auch die Rektal- oder Pharynxschleimhaut befallen. Bei hämatogener Aussaat können sie Arthritiden und selbst Endokarditiden verursachen. Rekurrierende Infektionen sind häufig. Dies hängt möglicherweise mit der Fähigkeit der Gonokokken zur Produktion einer IgA-Protease zusammen, die die sekretorischen Antikörper in den Schleimhautsekreten zerstört. Als Therapie erfolgt Antibiotikabehandlung, wobei das Antibiotikum der Wahl Penicillin G ist, das einzeitig oder, bei komplizierten Fällen, mehrzeitig gegeben wird. Alternative Substanzen sind Spectinomycin, ein penicillinasefestes Cephalosporin (z.B. Ceftriaxon) oder ein Tetracyclin. Diese Alternativen kommen bei Penicillinallergie oder bei penicillaseproduzierenden Stämmen zum Einsatz. Penicillasebildende Stämme breiten sich immer mehr aus.

Meningokokken (Neisseria meningitidis) sind Parasiten des Nasopharynx. 5 bis 10 % der Bevölkerung stellen Keimträger dar. Siedeln virulente Meningokokken auf der Schleimhaut und fehlen Antikörper beim Wirt, so kann es zum Eindringen in den Makroorganismus und zum Entstehen einer Bakteriämie kommen. Der Prädilektionsort für eine sekundäre Ansiedlung der Erreger ist zweifellos das Zentralnervensystem. Die Erreger können aber auch im Anschluß an eine Bakteriämie die Lunge, das Endokard oder die großen Gelenke befallen. Die Meningitis beginnt meist schlagartig, nach einer Inkubation von 2 bis 3 Tagen mit starken Kopfschmerzen, Fieber, Genickstarre und schwerem Krankheitsgefühl. Es handelt sich immer um eine Allgemeininfektion. Manchmal kann es zur schweren, mit Hämorrhagien einhergehenden Sepsis kommen (Waterhouse-Friderichsen-Syndrom). Als Therapie ist Penicillin G intravenös das Vorgehen der Wahl. Für Chloramphenicol spricht die gute Diffusion in den Liquorraum. Bei akuter Meningitis mit unbekanntem Erreger hat sich die Kombination Ampicillin/Chloramphenicol bewährt. In letzter Zeit wurden sehr gute Erfahrungen mit Cephalosporinen der dritten Generation, vor allem dem Ceftriaxon, gemacht. Der Vorteil dieser Antibiotika besteht darin, daß sie ein breites Spektrum aufweisen und deshalb auch gegen andere Meningitiserreger wirken. Wichtig ist ein frühzeitiges Einsetzen der Therapie, um Spätschäden zu verhüten. Meningokokkeninfekte treten oft gehäuft in Winter- und Frühlingsmonaten auf. Die Übertragung erfolgt durch Tröpfchen. Der Mensch ist das einzige Erregerreservoir. Infektionsquellen sind Keimträger und Erkrankte. Heute tritt die Infektion in den entwickelten Ländern in der Regel sporadisch auf. In den Ländern der Dritten Welt kann sie oft in Form von größeren Epidemien vorkommen. Die Mehrzahl der sporadischen Fälle ereignet sich im Kleinkindesalter. Die Inzidenz beträgt 2 bis 3 pro 100.000 pro Jahr. Unbehandelt beträgt die Letalität 85 %, bei rechtzeitiger Behandlung liegt sie unter 1 %.

Bisher bekannte lokal eingesetzte Antiseptika (z.B. Silbernitrat) besitzen eine sehr starke gewebetoxische Wirkung.

Es besteht daher nach wie vor der Bedarf nach geeigneten Mitteln, mit denen diese schwer zu therapierenden Infektionen wirksam behandelt werden können.

Die Aufgabe der vorliegenden Erfindung ist es daher, ein Mittel bereitzustellen, mit dem die durch sich intrazellulär vermehrende Erreger verursachten Infektionen wirksamer und schneller therapiert werden können. Insbesondere soll ein Mittel bereitgestellt werden, das lokal einsetzbar ist und eine hohe Wirksamkeit gegen sich intrazellulär vermehrende Erreger besitzt, ohne daß die Zellen des befallenen Gewebes zerstört werden.

Erfindungsgemäß wurde überraschend gefunden, daß diese Aufgabe durch die Verwendung von Poly(hexamethylen)biguanid mit einer mittleren Molekulargewichtsverteilung bis 5.000 gelöst werden kann.

Besonders bevorzugt ist ein Poly(hexamethylen)biguanid mit einer mittleren Molekulargewichtsverteilung von 1.000 bis 4.000 und insbesondere ein PHMB mit einer mittleren Molekulargewichtsverteilung von 1.700 bis 2.800. z.B. mit einem mittleren Molekulargewicht von 2.600.

Die Molekulargewichtsbestimmung erfolgt auf viskosimetrischem Wege.

Die Herstellung des erfindungsgemäß verwendeten Poly(hexamethylen)biguanids erfolgt in an sich bekannter Weise, z.B. wie es in der DE-PS 16 20 938 oder der GB-PS 1,202,495, auf deren Offenbarung hiermit im vollen Umfang Bezug genommen wird, beschrieben ist. Aus dem erhaltenen Poly(hexamethylen)biguanid kann gegebenenfalls in an sich bekannter Weise der unerwünschte Molekulargewichtsanteil, z.B. durch Dialyse. Molekularfiltration, HPLC, Gel-Permeations-Chromatographie (GPC), fraktionierte Fällung und dergleichen abgetrennt werden.

Erfindungsgemäß geeignet sind handelsübliches PHMB, wie z.B. Vantocil® IB oder Arlagard® E oder andere Handelsprodukte.

Das erfindungsgemäß eingesetzte PHMB liegt in freier Form oder in Form eines wasserlöslichen Salzes. z.B. als Hydrochlorid, als Pulver (z.B. gefriergetrocknet) in 100 %iger Konzentration oder in wässriger Lösung vor. Es ist in Konzentrationen bis 40 Gew.-%, z.B. in 2 bis 40 Gew.-%, vorzugsweise 3 bis 30 Gew.-%, insbesondere 4 bis 20 Gew.-% z.B. 4 Gew.-%, 4.5 Gew.-%, 5 Gew.-%. 6 Gew.-% in der wässrigen Lösung (d.h. als Konzentrat) verfügbar.

Unter "PHMB" wird hierin sowohl das Poly(hexamethylen)biguanid als auch das Poly(hexamethylen)biguanid in Form eines wasserlöslichen Salzes verstanden.

Die Konzentration, in der das erfindungsgemäß verwendete PHMB bzw. die wässrige Lösung des PHMB zur Behandlung von durch sich intrazellulär vermehrende Erreger verursachte Infektionen verwendet wird, hängt von dem beabsichtigten Verwendungszweck ab. Geeignete Konzentrationen liegen im allgemeinen im Bereich von 0,0001 bis 0,1 Gew.-%, vorzugsweise im Bereich von 0,0005 bis 0,05 Gew.-%, insbesondere im Bereich von 0,001 bis 0,04 Gew.-%, beispielsweise 0,04 Gew.-% (bezogen auf PHMB).

Das erfindungsgemäß verwendete PHMB kann zusammen mit die Oberflächenspannung herabsetzenden Tensiden, insbesondere augenverträglichen Tensiden, wie z.B. Polyethylengycol verwendet werden. Vorzugsweise wird Polyethylenglycol mit einem Molekulargewicht von 1.500 bis 6.000 und insbesondere ein solches Polyethylenglycol mit einem Molekulargewicht von 4.000, wie es unter dem Warenzeichen Lutrol® E 4000 von der Firma BASF AG vertrieben wird, angewandt. Das Verhältnis von PHMB zu dem Tensid liegt geeigneterweise im Bereich von 6:1 bis 24:1, vorzugsweise im Bereich von 12:1 bis 22:1 und beträgt insbesondere 20:1.

Das erfindungsgemäß verwendete PHMB ist gegenüber den schwierig zu therapierenden Infektionen, die durch sich intrazellulär vermehrende Erreger, wie Bakterien und Viren, hervorgerufen werden, in hohem Maße wirksam, ohne daß die Zellen des befallenen Gewebes zerstört werden. Besonders wird es zur Behandlung von durch sich intrazellulär vermehrende Bakterien wie Chlamydien. z.B. Chlamydia trachomatis oder Chlamydia psittaci, und Neisserien, z.B. Neisseria gonorrhoeae oder Neisseria memingitidis, insbesondere durch Chlamydien, wie Chlamydia trachomatis und Chlamydia psittaci verursachten Infektionen angewandt. Insbesondere erfolgt erfindungsgemäß die Behandlung der bisher schwierig zu therapierenden Infektionen im Auge und im Urogenitalbereich sowie im Peritonealraum.

Die Behandlung erfolgt erfindungsgemäß lokal.

Abhängig von der vorgesehenen Behandlung kann die Verwendung z.B. in Form von Tropfen, von wässrigen Lösungen (gegebenenfalls mit einem Gehalt an lactatfreier Ringerlösung oder Kochsalzlösung, vorzugsweise lactatfreier Ringerlösung). Emulsionen, Suspensionen, Gelen, Salben oder Cremes erfolgen, insbesondere in Form von Tropfen und Lösungen, gegebenenfalls mit einem Gehalt an lactatfreier Ringerlösung oder Kochsalzlösung, vorzugsweise lactatfreier Ringerlösung (in Form von Spüllösungen). In diesen Zubereitungsformen sind gegebenenfalls zusätzlich die zur Herstellung der jeweiligen Zubereitungsform notwendigen üblichen Hilfs- und Zusatzstoffe enthalten. Die Kochsalzlösung kann als 0,4 bis 1,2 Gew.-%ige Lösung eingesetzt werden. Vorzugsweise wird jedoch physiologische, d.h. 0,9 %ige Kochsalzlösung angewandt.

Das erfindungsgemäße PHMB kann sowohl zur Therapie als auch zur Prophylaxe sowie zur Kombinationstherapie mit Antibiotika, wie z.B. Tetracycline und Makrolid-Antibiotika, wie Erythromycin, bei Infektionen, die durch sich intrazellulär vermehrende Erreger, wie Chlamydien und Neisserien, insbesondere Chlamydien, verursacht werden, eingesetzt werden.

Die nachfolgenden Beispiele dienen der Erläuterung der vorliegenden Erfindung.

### Beispiel 1

Aus Poly(hexamethylen)biguanid mit einem mittleren Molekulargewicht M_{w} von 2.600, Polyethylenglycol 4.000 (Lutro® E 4000, BASF AG) und Wasser wurde durch Vermischen derselben eine Lösung der folgenden Zusammensetzung hergestellt:
- Poly(hexamethylen)biguanid-Hydrochlorid, M_{w} 2.600: 6 Gew.-%
- Polyethylenglycol, M_{w} 4.000: 0,3 Gew.-%
- Wasser: 93,7 Gew.-%.

Die hergestellte Lösung wurde nach entsprechender Verdünnung erfolgreich zur Behandlung von durch Chlamydien, wie Chlamydia trachomatis und Chlamydia psittaci verursachte Infektionen im Auge und im Urogenitalbereich angewandt.

Als Poly(hexamethylen)biguanid-Hydrochlorid wurde das handelsübliche Poly(hexamethylen)biguanid-Produkt Vantocil® IB oder Arlagard® E der Firma ICI eingesetzt. Vantocil® IB bzw. Arlagard® E ist eine wässrige Lösung, die als Wirkstoff 20 % Poly(hexamethylen)biguanid--Hydrochlorid enthält.

### Beispiel 2

Zur Herstellung einer weiteren Lösung für die erfindungsgemäße Verwendung wurde Beispiel 1 wiederholt mit der Ausnahme, daß anstelle des PHMB-Hydrochlorids das entsprechende PHMB eingesetzt wurde.

### Beispiel 3

Prüfung der Wirksamkeit von PHMB, M_{w} 2.800, gegenüber Chlamydia trachomatis.

Die Untersuchung wurde unter Verwendung einer wässrigen Lösung, die 0,02 Gew.-% PHMB, M_{w} 2.800, zusammen mit 0,001 Gew.-% Polyethylenglycol 4.000 enthielt, in folgender Weise durchgeführt:

Die Erregersuspension (Erreger: Chlamydia trachomatis) wurde mit der vorstehend genannten wässrigen Lösung (PHMB 0,02 %) für die Dauer der jeweiligen Einwirkzeit versetzt. Anschließend wurden die Erreger zentrifugiert, der Überstand wurde dekandiert, das Sediment wurde mit Zellkulturmedium versetzt und die Zentrifugation wurde wiederholt. Als Zellkulturmedium wurde Minimum Essential Medium (Eagle) mit Earles Salzen (Eagle. H., Science, 130, 342 (1959): Parker, R. C., Methods of Tissue Culture, 3rd Ed., HArper and Tow. New York) und 5 % fötalem Kälberserum, ohne Antibiotika, verwendet. Anschließend wurde das Sediment im Zellkulturmedium resuspendiert und die so gewaschene Erregersuspension wurde auf der Zellkultur (BGM-Zellen = Buffalo Green Monkey Zellen) titriert. Die Zellkulturen wurden fünf Tage bei 37°C bebrütet. Danach erfolgte die Bestimmung des Titers nach REED und MUENCH (vgl. Bonin, O., Quantitativ-virologische Diagnostik, Thieme, Stuttgart, 1973) durch mikroskopischen Nachweis von Chlamydieneinschlüssen in den BGM-Zellen nach Gimenez-Färbung (vgl. Balows et al. (ed), Manual of Clinical Microbiology, American Society for Microbiology, Washington DC, 1991. 1305).

Die Kontrolluntersuchung wurde in gleicher Weise, jedoch unter Verwendung von 0.9 Gew.-%iger NaCl-Lösung durchgeführt.

Die bei den Untersuchungen erhaltenen Ergebnisse sind nachfolgend in Tabelle I zusammengestellt:

**Tabelle I**

| **PHMB 0,02** %; | | | | |
|---|---|---|---|---|
| Einwirkzeit: | 5 min | 15 min | 30 min | Kontrolle |
| Versuch 1: Titer: | 3,0 x 10⁴ | 4,6 x 10³ | 4,6 x 10³ | 2,1 x 10⁸ |
| Versuch 2: Titer: | 5,2 x 10³ | 4,7 x 10² | 4,7 x 10² | 4,7 X 10⁸ |
| TITER-REDUKTION: > 7,0 x 10² bzw. 9,0 x 10⁴ EBE* nach 5 min, 4,6 x 10⁴ bzw. 1,0 x 10⁶ EBE nach 15 min und 4,6 x 10⁴ bzw. 1,0 x 10⁶ EBE nach 30 min. | | | | |

| | | | | |
|---|---|---|---|---|
| * EBE = Einschluß-bildende Einheiten | | | | |

Wie die erhaltenen Ergebnisse zeigen, ist das untersuchte PHMB in der Gebrauchsverdünnung von 0,02 % gegenüber Chlamydia trachomatis hochwirksam. Bei der Konzentration von 0,02 % erfolgte eine Reduktion der Infektiosität der Erregersuspension von 4 log₁₀-Stufen (99,99 %) bereits nach 5minütiger Einwirkungszeit und eine Reduktion von 6 log₁₀-Stufen nach 30minütiger Einwirkungszeit.

### Beispiel 4

Prüfung der Wirksamkeit von PHMB, M_{w} 2.800 gegenüber Chlamydia trachomatis.

Die Untersuchung gemäß Beispiel 3 wurde wiederholt mit der Ausnahme, daß anstelle der in Beispiel 3 verwendeten wässrigen Lösung (0,02 Gew.-% PHMB) eine Lösung verwendet wurde, die 0,04 Gew.-% PHMB zusammen mit 0,002 Gew.-% Polyethylenglycol 4.000 enthielt.

Die bei dieser Untersuchung erhaltenen Ergebnisse sind in der nachfolgenden Tabelle II zusammengestellt.

**Tabelle II**

| **PHMB 0,04 %**; | | | | |
|---|---|---|---|---|
| Einwirkzeit: | 5 min | 15 min | 30 min | Kontrolle |
| Versuch 1: Titer: | 3,2 x 10² | 3,2 x 10² | 3,2 x 10² | 4,7 x 10⁸ |
| Versuch 2: Titer: | 3,2 x 10² | 3,2 x 10² | 3,2 x 10² | 1,0 x 10⁹ |
| TITER-REDUKTION: 1,5 bis 3,1 x 10⁶ EBE* nach 5, 15 und 30 min. | | | | |

| | | | | |
|---|---|---|---|---|
| *EBE = Einschluß-bildende Einheiten | | | | |

Wie aus den vorstehend zusammengestellten Ergebnissen ersichtlich, ist das untersuchte PHMB bei der Gebrauchsverdünnung von 0,04 Gew.-% gegenüber Chlamydia trachomatis hochwirksam. Unter den getesteten Bedingungen erfolgte bei einer Konzentration des PHMB von 0,04 Gew.-% eine Verminderung der Infektiosität der Erregersuspension um mindestens 6 log₁₀-Stufen (99,9999 %) bereits nach 5minütiger Einwirkungszeit.

### Beispiel 5

Prüfung der Wirksamkeit von PHMB, M_{w} 2.600, gegenüber Chlamydia psittaci.

Die Untersuchung wurde unter Verwendung einer wässrigen Lösung, die 0,02 Gew.-% PHMB. M_{w} 2.600, zusammen mit 0,001 Gew.-% Polyethylenglycol 4.000 enthielt, in der gemäß Beispiel 3 beschriebenen Weise durchgeführt.

Die Kontrolluntersuchung wurde in gleicher Weise, jedoch unter Verwendung von 0,9 Gew.-%iger NaCl-Lösung durchgeführt.

Die bei den Untersuchungen erhaltenen Ergebnisse sind nachfolgend in Tabelle III zusammengestellt.

**Tabelle III**

| **PHMB 0,02 %**; | | | | |
|---|---|---|---|---|
| Einwirkzeit: | 5 min | 15 min | 30 min | Kontrolle |
| Versuch 1: Titer: | 3,2 x 10⁵ | 2,0 x 10⁴ | 3,2 x 10⁴ | 3,2 x 10⁸ |
| Versuch 2: Titer: | 1,0 x 10⁷ | 3,2 x 10⁶ | 3,2 x 10⁶ | 3,2 x 10⁹ |
| TITER-REDUKTION: 1,0 x 10³ bzw. 3,2 x 10² EBE* nach 5 min, 1,6 x 10⁴ bzw. 1,0 x 10³ EBE nach 15 min 1,0 x 10⁴ bzw. 1,0 x 10³ EBE nach 30 min. | | | | |

| | | | | |
|---|---|---|---|---|
| *EBE = Einschluß-bildende Einheiten | | | | |

Aus den vorstehenden Ergebnissen ist ersichtlich, daß das untersuchte PHMB in der Gebrauchsverdünnung von 0,02 % gegenüber Chlamydia psittaci wirksam ist. Unter den getesteten Bedingungen erfolgte eine Verminderung der Infektiosität der Erregersuspension um ≥ 3 log₁₀-Stufen (99,9 %) bereits nach fünf Minuten Einwirkungszeit.

### Beispiel 6

Prüfung der Wirksamkeit von PHMB, M_{w} 2.600 gegenüber Chlamydia psittaci.

Die Untersuchung gemäß Beispiel 5 wurde wiederholt mit der Ausnahme, daß anstelle der in Beispiel 5 verwendeten wässrigen Lösung (0,02 Gew.-% PHMB) eine Lösung verwendet wurde, die 0,04 Gew.-% PHMB zusammen mit 0,002 Gew.-% Polyethylenglycol 4.000 enthielt.

Die bei dieser Untersuchung erhaltenen Ergebnisse sind in der nachfolgenden Tabelle IV zusammengestellt.

**Tabelle IV**

| **PHMB 0,04 %**; | | | | |
|---|---|---|---|---|
| Einwirkzeit: | 5 min | 15 min | 30 min | Kontrolle |
| Versuch 1: Titer: | 2,2 x 10⁵ | 4,7 x 10³ | 4,7 x 10³ | 3,2 x 10⁸ |
| Versuch 2: Titer: | 2,2 x 10⁴ | 2,1 x 10³ | 1,0 x 10³ | 1,0 x 10⁹ |
| TITER-REDUKTION: 1,5 x 10³ bzw. 4,6 x 10⁴ EBE* nach 5 min. 6,8 x 10⁴ bzw. 4,6 x 10⁵ EBE nach 15 min 6,8 x 10⁴ bzw. 1,0 x 10⁶ EBE nach 30 min. | | | | |

| | | | | |
|---|---|---|---|---|
| *EBE = Einschluß-bildende Einheiten | | | | |

Aus den erhaltenen Ergebnissen ist ersichtlich, daß das untersuchte PHMB in der Konzentration von 0,04 % gegenüber Chlamydia psittaci wirksam ist. Unter den getesteten Bedingungen erfolgte eine Verminderung der Infektiosität der Erregersuspension um ≥ 3 log₁₀-Stufen (99,9 %) bereits nach fünfminütiger Einwirkzeit der Prüfsubstanz. Bereits nach 15 Minuten Einwirkzeit wurde bei Anwendung dieser Gebrauchsverdünnung (0,04 %) im Mittel eine Reduktion von > 10⁵ EBE und nach 30 Minuten im Mittel eine Reduktion von > 5 x 10⁵ EBE erzielt.

### Beispiel 7

Prüfung der Wirksamkeit von PHMB, M_{w} 2.600 gegenüber Neisseria gonorrhoeae.

Das PHMB, M_{w} 2.600 wurde in den nachfolgend in Tabelle V angegebenen Konzentrationen gemäß den Richtlinien für die Prüfung und Bewertung chemischer Desinfektionsverfahren (Stand vom 01.01.1981) der Deutschen Gesellschaft für Hygiene und Mikrobiologie (DGHM), in modifizierter Form, untersucht.
Anzuchtmedium: Thayer-Martin-Medium (Publ. Health Rep., 81, S. 559-562 (1966) und 82. S. 361-363 (1967), Publ. Health Lab. J., 22, 104-109 (1964), Ärzt. Lab., 22, 60-65 (1976) aus Agar-Basis mit Hämoglobinpulver und Selektiv-Supplement.
Inaktivierungsmedium: 3 % Tween + 3 % Saponin + 0,1 % Histidin + 0,1 % Cystein (Herstellung des Inaktivierungsmediums ist in den Richtlinien der DGHM vom 1.1.1981 beschrieben).
Bebrüten der Neisserien Subkulturen: 72 Stunden bei 37°C in CO₂-Atmosphäre.

Die bei dieser Untersuchung erhaltenen Ergebnisse sind in der nachfolgenden Tabelle V zusammengestellt.

**Tabelle V**

| Konzentration (%) PHMB | Reduktionsfaktoren (log) nach Einwirkzeit in Minuten | | | |
|---|---|---|---|---|
| | 1 | 2 | 5 | 15 |
| Ohne Albuminbelastung | | | | |
| 0,0125 | 2,69 | ≥ 3,76 | ≥ 3,70 | ≥ 3,60 |
| 0,00625 | 2,53 | ≥ 3.76 | ≥ 3,70 | ≥ 3,60 |
| 0,003125 | 2,36 | ≥ 3,76 | ≥ 3,70 | ≥ 3,60 |
| 0,000625 | 2,21 | ≥ 3,76 | ≥ 3,70 | ≥ 3,60 |
| Kontrollwert log ohne PHMB | 4,83 | 4,76 | 4,70 | 4,60 |

| Mit 0,2 % Albuminbelastung | | | | |
|---|---|---|---|---|
| 0,0125 | 2,04 | 2,79 | ≥ 3,68 | ≥ 3,62 |
| 0,00625 | 1,74 | 2,32 | ≥ 3,68 | ≥ 3,62 |
| 0,003125 | 1,36 | 2,24 | 2,38 | ≥ 3,62 |
| 0,000625 | 0,33 | 0,58 | 1,66 | 1,73 |
| Kontrollwert log ohne PHMB | 4,82 | 4,79 | 4,68 | 4,62 |

Aus den vorliegenden Ergebnissen ist ersichtlich, daß im quantitativen Suspensionstest durch die 0,00625 %ige Konzentration von PHMB ohne und mit Eiweißbelastung (Albumin) eine logarithmische Reduktion von Neisseria gonorrhoeae über die Nachweisgrenze des Versuchs hinaus von ≥ 3,70 bereits nach 5 Minuten verursacht wurde. Derselbe Effekt wurde durch eine 0,003125 %ige Konzentration nach 15 Minuten erzielt.

### Beispiel 8

Prüfung der Wirksamkeit von PHMB 4.000 (Polyhexamethylenbiguanidhydrochlorid, mittlere Molekulargewichtsverteilung 4.000) gegenüber Neisseria gonorrhoeae

Das PHMB 4.000 wurde in den nachfolgend, in Tabelle VI angegebenen Konzentrationen gemäß den Richtlinien für die Prüfung und Bewertung chemischer Desinfektionsverfahren (Stand vom 01.01.1981) der Deutschen Gesellschaft für Hygiene und Mikrobiologie, in modifizierter Form, untersucht.
Anzuchtmedium: Thayer-Martin-Medium aus Agar-Basis mit Hämoglobinpulver und Selektiv-Supplement (vgl. Beispiel 7).
Inaktivierungsmedium: 3 % Tween + 3 % Saponin + 0,1 % Histidin + 0,1 % Cystein.
Bebrüten der Neisserien Subkulturen: 72 Stunden bei 37°C in CO₂-Atmosphäre.

Die bei dieser Untersuchung erhaltenen Ergebnisse sind in der nachfolgenden Tabelle VI zusammengestellt.

**Tabelle VI**

| Konzentration (%) PHMB 4.000 | Reduktionsfaktoren (log) nach Einwirkzeit in Minuten | | | |
|---|---|---|---|---|
| | 1 | 2 | 5 | 15 |
| Ohne Albuminbelastung | | | | |
| 0,003 | 2,45 | ≥ 3,74 | ≥ 3,65 | ≥ 3,54 |
| 0,0015 | 2,34 | ≥ 3,74 | ≥ 3,65 | ≥ 3,54 |
| 0,00075 | 2,15 | ≥ 3,74 | ≥ 3,65 | ≥ 3,54 |
| 0,00015 | 1,95 | ≥ 3,74 | ≥ 3,65 | ≥ 3,54 |
| Kontrollwert log ohne PHMB | 4,80 | 4,74 | 4,65 | 4,54 |

| Mit 0,2 % Albuminbelastung | | | | |
|---|---|---|---|---|
| 0,003 | 1,85 | 2,43 | ≥ 3,72 | ≥ 3,68 |
| 0,0015 | 1,56 | 2,11 | ≥ 3,72 | ≥ 3,68 |
| 0,00075 | 1,15 | 2,01 | 2,04 | ≥ 3,68 |
| 0,00015 | 0,25 | 0,49 | 1,57 | 1,67 |
| Kontrollwert log ohne PHMB | 4,85 | 4,81 | 4,72 | 4,68 |

Aus den vorliegenden Ergebnissen ist ersichtlich, daß im quantitativen Suspensionstest durch die 0,0015 %ige Konzentration von PHMB 4.000 ohne und mit Eiweißbelastung (Albumin) eine logarithmische Reduktion von Neisseria gonorrhoeae über die Nachweisgrenze des Versuchs hinaus von ≥ 3.65 bereits nach 5 Minuten verursacht wurde. Derselbe Effekt wurde durch eine 0,00075 %ige Konzentration nach 15 Minuten erzielt.

### Beispiel 9

### Toleranztest

Eine wässrige Lösung mit einem Gehalt an
20 % PHMB, M_{w} 2.600 und
1 % Polyethylenglycol, M_{w} 4.000
wurde mit Ringerlösung (ohne Lactat) auf eine Konzentration von 0,2 % verdünnt (entspricht einer Konzentration an PHMB von 0,04 Gew.-%) und einem 4wöchigen Toleranztest unterworfen.

Als Kontrollösung wurde 0,9 %ige Natriumchloridlösung angewandt.

Als Testtiere wurden 24 (pigmentierte) Himalaya-Kaninchen (12 männliche und 12 weibliche Tiere) verwendet, die in drei Gruppen zu je 4 Tieren pro Geschlecht eingeteilt wurden. Die Verabreichung erfolgte auf zwei Wegen:
a) Instillation in den Konjunktivalsack des rechten Auges (das linke Auge bleibt unbehandelt), wobei 50 µl pro Konjunktivalsack instilliert wurden,
b) Anwendung in die Vagina, wobei 2 ml pro Vagina angewandt wurden.

Dauer des Tests: 20tägige Anpassung und 4wöchige Behandlung.

Die Testbedingungen in den einzelnen Gruppen waren folgende:

### Gruppe 1: Kontrollgruppe

- Auge:: 50 µl 0,9 %ige Natriumchloridlösung, 8 Instillationen pro Tier und Tag in 45minütigen Intervallen,
- Vagina:: 2 ml 0,9 %ige Natriumchloridlösung, 3 Instillationen pro Tier und Tag in 3stündigen Intervallen,

### Gruppe 2:

- Auge:: 50 µl der 0,2 %igen Testlösung, 2 Instillationen pro Tier und Tag in einem 6stündigen Intervall.
- Vagina:: 2 ml der 0,2 %igen Testlösung, 2 Instillationen pro Tier und Tag in einem 6stündigen Intervall.

### Gruppe 3:

- Auge:: 50 µl der 0,2 %igen Testlösung, 8 Instillationen pro Tier und Tag in 45minütigem Intervall,
- Vagina:: 2 ml der 0,2 %igen Testlösung, 3 Instillationen pro Tier und Tag in 3stündigen Intervallen.

Bei diesen Untersuchungen wurden folgende Ergebnisse erhalten:

### Untersuchung des Auges:

Bei 8maligen Instillationen von je 50 µl der 0,2 %igen Testlösung pro Tag über einen Zeitraum von 4 Wochen in den Konjunktivalsack des rechten Auges wurde eine milde, vorübergehende Konjunktivalrötung am 1., 2. und/oder 3. Testtag verursacht. Bei einer zweimaligen Instillation von je 50 µl der 0,2 % igen Testlösung pro Tag über einen Zeitraum von 4 Wochen wurde kein derartiger Effekt beobachtet. Es traten keinerlei Änderungen auf. Die Histopathologie des Auges ergab keinerlei substanzbezogenen Änderungen.

### Untersuchung der Vigina:

Die zwei- oder dreimalige Verabreichung von je 2 ml der 0,2 %igen Testlösung pro Tag über einen Zeitraum von 4 Wochen in die Vagina führte zu keinen substanzbezogenen lokalen Intoleranzreaktionen.

### Mortalität:

Keines der Tiere starb vorzeitig.

### Klinische Symptome:

Es traten keine auf die Behandlung bezogenen klinischen Symptome auf.

### Körpergewicht:

Die Körpergewichte lagen im Normalbereich.

### Nahrungsaufnahme:

Es wurde kein substanzbezogener Einfluß hinsichtlich der Nahrungsaufnahme beobachtet.

Ferner wurde postmortem bei keinem der Kaninchen behandlungsbezogene pathologische Veränderungen oder irgendein substanzbezogener Einfluß auf die absoluten und relativen Organgewichte gefunden.

## Patentansprüche

1. Verwendung von Poly(hexamethylen)biguanid mit einer mittleren Molekulargewichtsverteilung bis 5.000 zur Behandlung von durch sich intrazellulär vermehrende Bakterien verursachte Infektionen.

2. Verwendung nach Patentanspruch 1, **dadurch gekennzeichnet**, daß das Poly(hexamethylen)biguanid eine mittlere Molekulargewichtsverteilung von 1.000 bis 4.000 besitzt.

3. Verwendung nach Patentanspruch 2, **dadurch gekennzeichnet**, daß das Poly(hexamethylen)biguanid eine mittlere Molekulargewichtsverteilung von 1.700 bis 2.800 besitzt.

4. Verwendung nach einem der Patentansprüche 1 bis 3, **dadurch gekennzeichnet**, daß das Poly(hexamethylen)biguanid zusammen mit einem die Oberflächenspannung erniedrigenden Tensid verwendet wird.

5. Verwendung nach Patentanspruch 4, **dadurch gekennzeichnet**, daß das Tensid Polyethylenglycol ist.

6. Verwendung nach Patentanspruch 5, **dadurch gekennzeichnet**, daß das Verhältnis von Poly(hexamethylen)biguanid zu Polyethylenglycol im Bereich von 6:1 bis 24:1 liegt.

7. Verwendung nach Patentanspruch 6, **dadurch gekennzeichnet**, daß das Verhältnis im Bereich von 12:1 bis 22:1 liegt.

8. Verwendung nach einem oder mehreren der Patentansprüche 1 bis 7, **dadurch gekennzeichnet**, daß das Poly(hexamethylen)biguanid in lactatfreier Ringerlösung oder Kochsalzlösung angewandt wird.

9. Verwendung nach einem oder mehreren der Patentansprüche 1 bis 8, **dadurch gekennzeichnet**, daß das Poly(hexamethylen)biguanid in einer Konzentration von 0,0001 bis 0,1 Gew.-% angewandt wird.

10. Verwendung nach Patentanspruch 9, **dadurch gekennzeichnet**, daß die Konzentration 0,0005 bis 0,05 Gew.-% beträgt.

11. Verwendung nach Patentanspruch 10, **dadurch gekennzeichnet**, daß die Konzentration 0,001 bis 0,04 Gew.-% beträgt.

12. Verwendung nach einem oder mehreren der Patentansprüche 1 bis 11 zur Behandlung von durch Chlamydien und/oder Neisserien verursachte Infektionen.

13. Verwendung nach einem oder mehreren der Patentansprüche 1 bis 12 zur Behandlung von Infektionen im Auge.

14. Verwendung nach einem oder mehreren der Patentansprüche 1 bis 12 zur Behandlung von Infektionen im Urogenitaltrakt.

15. Verwendung nach einem oder mehreren der Patentansprüche 1 bis 12 zur Behandlung von Infektionen im Peritonealraum.
